# EUROPÄISCHE PATENTSCHRIFT

(11) **EP 0 744 990 B1**
(45) Veröffentlichungstag und Bekanntmachung des Hinweises auf die Patenterteilung: **29.10.1997**
(21) Anmeldenummer: 95908216.5
(22) Anmeldetag: 27.01.1995
(51) Int. Cl.: B01D 53/86, B01D 53/72, A61L 2/20

(54) **VERFAHREN ZUR ABSCHEIDUNG VON ETHYLENOXID AUS ABLUFT- ODER ABGASSTRÖMEN**
PROCESS FOR REMOVING ETHYLENE OXIDE FROM OUTGOING AIR OR EXHAUST GAS STREAMS
PROCEDE D'ELIMINATION DE L'OXYDE D'ETHYLENE CONTENU DANS DES COURANTS D'AIR EVACUE OU DE GAZ D'ECHAPPEMENT

(30) Priorität: 19.02.1994 DE 4405276
(43) Veröffentlichungstag der Anmeldung: 04.12.1996
(73) Patentinhaber: OTTO LUFT- und KLIMATECHNIK GmbH & Co. KG, 57319 Bad Berleburg (DE)
(72) Erfinder: FATTINGER, Volker, CH-4144 Arlesheim (CH); HABERLAND, Hans, D-64625 Bensheim 3 (DE)
(74) Vertreter: Katscher, Helmut, Dipl.-Ing.
(86) Internationale Anmeldenummer: EP9500306
(87) Internationale Veröffentlichungsnummer: WO9522396

(56) Entgegenhaltungen:
- WO-A-92/20432
- DE-A- 2 333 574
- DE-A- 3 800 380
- DE-A- 3 835 161
- US-A- 1 954 395

## Beschreibung

Die Erfindung betrifft ein Verfahren zur Abscheidung von Ethylenoxid aus Abluft- oder Abgasströmen, wobei unter Einsatz einer Säure als Katalysator aus Ethylenoxid und Wasser Glycol gebildet wird.

Ethylenoxid wird in der pharmazeutischen Industrie, im Krankenhausbereich und auch in der Lebensmittelindustrie zur Sterilisation von temperaturempfindlichen medizinischen Instrumenten, Pharmaka, Packstoffen, Gewürzen usw. eingesetzt. Gemäß den Erkenntnissen bezüglich der kanzerogenen Wirkung wurde Ethylenoxid in Deutschland in die Klasse III der Schadstofflisten eingestuft. Abluftströme aus Sterilisationskammern sind relativ klein, meist im Bereich von 10 bis 500 Nm³/h.

Es ist bekannt, die Abluft aus Sterilisationskammern mittels Gaswaschanlagen zu reinigen. Als Waschflüssigkeit dient z. B. eine 1-5 %-ige Schwefelsäure. Die Schwefelsäure (oder auch andere Säuren) katalysiert die Reaktion von Ethylenoxid mit Wasser unter Bildung von Monoethylenglycol. Es entstehen aber auch andere Reaktionsprodukte, wie Diethylenglycol, Triethylenglycol und in geringen Mengen auch höhere Ethylenglycole. Im Zusammenhang mit der Erfindung wird das Reaktionsprodukt aus Ethylenoxid und H₂O als Glycol bezeichnet.

Wegen des ungünstigen Phasengleichgewichts mit Wasser und der verhältnismäßig langsamen Umsetzung zu Glycol erfordert das Auswaschen des Ethylenoxids auf Werte von weniger als 5 mg/Nm³ relativ teure Waschanlagen.

Nach der Entnahme aus der Sterilisationskammer haftet noch Ethylenoxid an den sterilisierten Gegenständen. Die Lagerräume für die sterilisierten Güter müssen daher belüftet werden um Ethylenoxid auszuspülen. Dabei entstehen relativ große Abluftströme im Bereich von z. B. 1000 bis 10 000 Nm³/h. Ein Auswaschen des Ethylenoxids auf Konzentrationen von weniger als 5 mg/Nm³ erfordert unverhältnismäßig große und teure Gaswaschsysteme. Ein Verbrennen des Ethylenoxids in den großen Luftmassen würde sehr hohe Brennstoffkosten verursachen.

Es ist auch bekannt, Abluft durch Adsorption von Ethylenoxid zu befreien. Hierzu wird z. B. speziell präparierte Aktivkohle verwendet. Dabei verursacht jedoch das Regenieren der Aktivkohle Probleme. Die Reaktionsprodukte des Ethylenoxids reichern sich auf der Aktivkohle an. Eine Desorption mittels Wärme und/oder Vakuum ist wegen des geringen Dampfdrucks der aus Ethylenoxid gebildeten Reaktionsprodukte und wegen der Gefahr der Verharzung nicht möglich. Das Reaktivieren von Kohle bei hoher Temperatur führt zu hohen Kosten und verursacht einen Verlust eines Teils der Aktivkohle.

Bei einem bekannten Verfahren (WO 92/20432) befindet sich das Adsorbensbett bei der Beladung in nassem Zustand. Die Umwandlung von Ethylenoxid in Glykol wird mindestens teilweise während der Regenerierung des beladenen Adsorbesnbetts in einer Mischung aus Wasser als Extaktionsflüssigkeit und Schwefelsäure als Katalysator ausgeführt. Eine Beladung des Adsorbensbetts mit Katalysator vor der Einleitung des Abgases findet nicht statt.

Bei einem anderen bekannten Verfahren (DE-A-2 33 574) kommt kein Adsorbens sondern ein Ionenaustauscher zum Einsatz.

Aufgabe der Erfindung ist es, das Entfernen des Ethylenoxids aus Abluft- oder Abgasströmen auf Konzentrationen unter der analytischen Nachweisbarkeit, insbesondere weit unter 1 mg/Nm³, in ökonomischer Weise zu ermöglichen.

Diese Aufgabe wird erfindungsgemäß mit den kennzeichnenden Merkmalen des Anspruchs 1 gelöst.

Die Abscheidung des Ethylenoxid erfolgt, indem der zu reinigende Abgas- bzw. Abluftstrom durch ein Bett eines Adsorbens geleitet wird, welches einen Katalysator für die Glycolbildung enthält.

Zur Regeneration des Adsorbens wird das Adsorbens mit einer Flüssigkeit extrahiert, welche das Glycol löst. Dem von Glycol befreiten Adsorbens wird neuer Katalysator zugesetzt und nach einem Trockenvorgang steht das Adsorbensbett wieder für die Abgasreinigung zur Verfügung.

Als geeignetes Adsorbens erwies sich z. B. Aktivkohle mit einer Korngröße von 3 bis 6 mm, welche 1 bis 5 % Säure enthält. Geeignet sind Säuren mit geringem Dampfdruck, z. B. Schwefelsäure. Zum Extrahieren des Glycols kann vorteilhaft heißes Wasser eingesetzt werden.

Die erfindungsgemäße adsorptive Ethylenoxid-Abscheidung ist insbesondere dann von Vorteil, wenn relativ geringe Ethylenoxid-Konzentrationen (z. B. weniger als 50 mg/Nm³) aus großen Abluftströmen entfernt werden müssen.

Bei höheren Ethylenoxid-Konzentrationen (z. B. mehr als 50 mg/Nm³) ist eine Gaswäsche zweckmäßig, um Restgehalte von ca. 15 mg/Nm³ zu erreichen. In einer vorteilhaften Ausgestaltung der Erfindung wird eine Gaswäsche als Vorreinigung eingesetzt. Die bei der Extraktion der beladenen Aktivkohle anfallende Flüssigkeit wird in dieser absorptiven Vorreinigung (Gaswäsche) eingesetzt.

In der vorgeschalteten Waschstufe wird die Ethylenoxid-haltige Abluft befeuchtet, so daß im nachgeschalteten Adsorbens-Bett das für die Glycolbildung als Reaktionspartner benötigte Wasser zur Verfügung steht. Die Verdunstung von Wasser in der Vorreinigung bewirkt eine Erhöhung des Glycolkonzentration in der Gaswaschflüssigkeit. Sobald eine genügende Konzentration (z. B. 35 Gewichts-%) erreicht ist, wird ein Teil der Waschflüssigkeit entnommen und z. B. mit Kalkmilch neutralisiert. Der gebildete Gips wird durch Filtration oder Zentrifugieren abgetrennt, und die neutrale Glycollösung kann einer Verwertung zugeführt werden. Es besteht aber auch die Möglichkeit, die Glycol-haltige Flüssigkeit einem biologischen Abbau, z. B. in einer Rieselbett-Biologie zuzuführen.

Die von Ethylenoxid befreite Abluft kann vorteilhaft zur Belüftung des biologischen Abbaus von Glycol herangezogen werden.

Das erfindungsgemäße Verfahren führt zu keinerlei Entsorgungsproblemen. Das Glycol wird von den Mikroorganismen zu CO₂ und zu H₂O abgebaut. Die bei der Neutralisation entstehenden geringen Mengen an Gips (CASO₄.2 H₂O) sind kein Gefahrstoff und problemlos zu entsorgen.

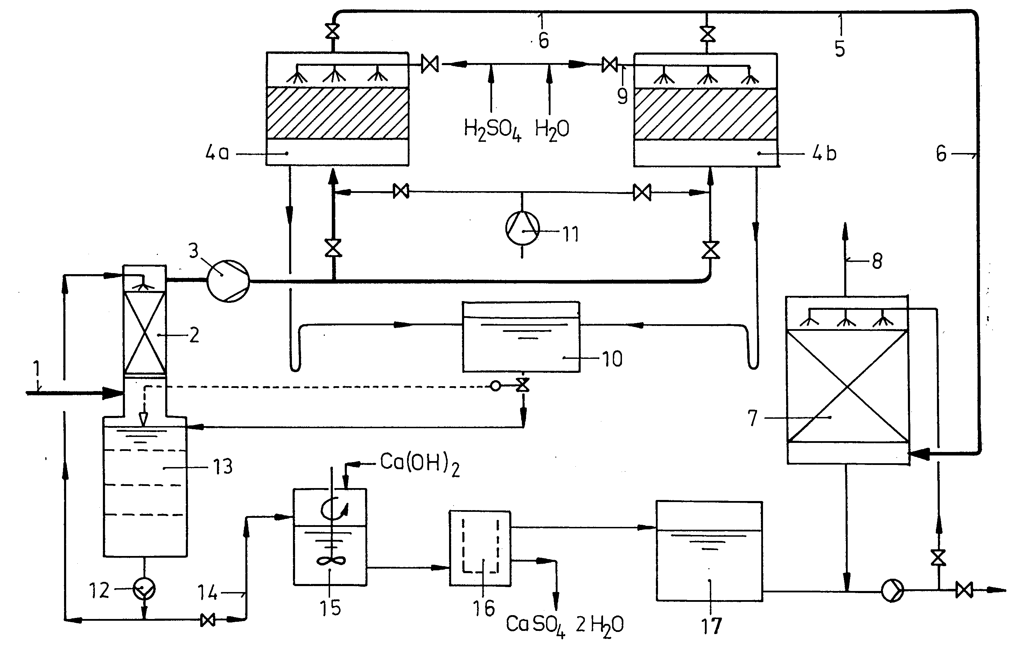

Nachfolgend wird ein Ausführungsbeispiel der Erfindung anhand der Zeichnung näher erläutert.

Die Zeichnung zeigt eine Abluftreinigungsanlage. Durch eine Gasleitung strömt ein Abluftstrom von 6000 Nm³/h mit 35°C in die schematisch dargestellte Anlage. Die Abluft stammt aus Lagerhallen für medizinische Instrumente, die mit Ethylenoxid sterilisiert wurden. Die Konzentration von Ethylenoxid liegt im Durchschnitt bei 45 mg/m³, jedoch treten zeitweise Spitzen bis zu 800 mg/Nm³ auf.

Der Gasstrom durchströmt eine Füllkörperschicht, deren Volumen 10 m³ beträgt. Ein Ventilator fördert den Abluftstrom von unten nach oben durch ein adsorptives Filter. Als Adsorbens dienen ca 5 m³ Aktivkohle einer Korngröße von 3 bis 6 mm. Die Aktivkohle enthält 2 Gewichts-% H₂SO₄. Versuche zeigten, daß anstelle von Aktivkohle auch körniges Silicagel verwendet werden kann, welches 2 % H₂SO₄ oder 3 % H₃PO₄ enthält.

Ein Abluftüberwachungsgerät registriert die Ethylenoxid-Konzentration im Reingas. Wird ein bestimmter Wert (z. B. 1 mg Ethylenoxid pro m³) überschritten, dann ist die Aufnahmekapazität des Filters 4a erschöpft, und durch entsprechende Steuerung der Absperrarmaturen wird ein Filter 4b eingeschaltet und das Filter 4a abgesperrt. Die Standzeit eines Filters beträgt ca. einen Monat. Der von Ethylenoxid befreite Abluftstrom gelangt durch eine Leitung 6 in einen biologischen Rieselbettreaktor 7 und durch eine Leitung 8 in die Atmosphäre.

Die Regeneration eines der beiden adorptiven Filter 4a oder 4b wird anhand des Filters 4b erläutert. Mittels einer Leitung 9 werden ca. 50 m³heißes Wasser, portionsweise im Verlauf mehrerer Tage, auf das Adsorbensbett gesprüht. Das heiße Wasser löst das Glycol und auch die im Adsorbens enthaltene H₂SO₄ und gelangt in einen Waschflüssigkeitstank 10. Nach Beendigung der Spülung des Adsorbensbettes wird eine gemessene Menge 20-50 %-iger Schwefelsäure auf das Adsorbens gesprüht, und danach wird zur Trocknung des Adsorbens mittels eines Ventilators 11 Luft durch das Adsorbens geblasen. Nach Beendigung der Trocknung wird der Ventilator 11 abgestellt, und das Filter 4b ist wieder betriebsbereit.

Eine Pumpe 12 dient zur Berieselung der Füllkörperschicht 2 mit ca. 30 m³/h Gaswaschflüssigkeit. Die aus der Füllkörperschicht 2 abfließende Flüssigkeit gelangt in einen Reaktionstank 13. Dieser Reaktionstank 13 enthält Lochblecheinbauten, die eine Kolbenströmung von oben nach unten sicherstellen. Bei einer Verweilzeit von ca. 30 Minuten reagiert das von der Waschflüssigkeit aufgenommene Ethylenoxid mit H₂O unter Bildung von Glycol. In der Füllkörperschicht 2 verdunstet Wasser.

Wie schematisch in der Zeichnung dargestellt, wird das Flüssigkeitsniveau im Reaktionstank 13 durch Zufluß von Flüssigkeit aus dem Waschflüssigkeitstank 10 konstantgehalten.

Durch chemische Umsetzung von Ethylenoxid zu Glycol und durch Zufuhr von Glycol mit der Waschflüssigkeit aus dem Tank 10 steigt die Glycolkonzentration laufend an. Sobald eine Konzentration von z. B. 40 % erreicht ist, wird mittels einer Leitung 14 Glycollösung in einen Rührbehälter 15 geleitet und dort mit Ca(OH)₂ neutralisiert. In einem Filter 16 wird der gebildete Gips abgetrennt, und die neutrale Glycollösung gelangt in einen Lagertank 17. Aus diesem Lagertank kann Glycollösung in Transportfahrzeuge gepumpt werden. Falls eine Verwertung nicht wirtschaftlich ist, wird das Glycol im biologischen Rieselbettreaktor 7 zu CO₂ und H₂O abgebaut.

## Patentansprüche

1. Verfahren zur Abscheidung von Ethylenoxid aus Abluft- oder Abgasströmen, wobei unter Einsatz eine Säure als Katalysator aus Ethylenoxid und Wasser Glycol gebildet wird, dadurch gekennzeichnet, daß
a) der zu reinigende, mit Wasser befeuchtete Gasstrom durch ein Bett eines Adsorbens geleitet wird, welches den Katalysator enthält,
b) eine Flüssigkeit zur Extraktion des Glycols aus dem Adsorbens verwendet wird und
c) das extrahierte Adsorbens getrocknet und mit Katalysator wieder beladen wird, bevor es zur weiteren Abgasreinigung Verwendung findet.

2. Verfahren nach Anspruch 1, dadurch gekennzeichnet, daß als Adsorbens körnige Aktivkohle verwendet wird.

3. Verfahren nach Anspruch 1, dadurch gekennzeichnet, daß als Katalysator Schwefelsäure eingesetzt wird.

4. Verfahren nach Anspruch 1, dadurch gekennzeichnet, daß als Extraktionsmittel Wasser verwendet wird.

5. Verfahren nach Anspruch 4, dadurch gekennzeichnet daß das Wasser durch die Adsorbens-Extraktion mit Glycol und Schwefelsäure beladen zur Vorreinigung der Ethylenoxid-haltigen Abluft eingesetzt wird.

6. Verfahren nach Anspruch 5, dadurch gekennzeichnet, daß reines Wasser, welches im Bett des Adsorbens mit Ethylenoxid unter Bildung von Glycol reagiert, durch Wasserverdunstung der Glycol-Lösung gewonnen wird, die aus der Vorreinigung der Abluft entsteht.

7. Verfahren nach Anspruch 6, dadurch gekennzeichnet, daß die durch Wasserverdunstung aufkonzentrierte Flüssigkeit mit Calcium-haltigen Chemikalien neutralisiert wird.

8. Verfahren nach Anspruch 7, dadurch gekenzeichnet, daß der gebildete Gips abgetrennt und die neutralisierte, Glycol-haltige Flüssigkeit einem biologischen Abbau unterworfen wird.

9. Verfahren nach Anspruch 8, dadurch gekennzeichnet, daß die von Ethylenoxid befreite Abluft zur Belüftung des biologischen Glycol-Abbaus herangezogen wird.

## Claims

1. Process for removing ethylene oxide from outgoing air or exhaust gas currents, where glycol is formed from ethylene oxide and water, using an acid as catalyst, characterised in that
a) the water-moistened gas stream to be cleaned is passed through a bed of adsorbent, containing the catalyst
b) a liquid is used to extract the glycol from the adsorbent and
c) the extracted adsorbent is dried and recharged with catalyst before being used again for cleaning exhaust gas.

2. Process according to claim 1, characterised in that granulated activated charcoal is used as the adsorbent.

3. Process according to claim 1, characterised in that sulphuric acid is used as the catalyst.

4. Process according to claim 1, characterised in that water is used as the extraction medium.

5. Process according to claim 4, characterised in that the water, charged with glycol and sulphuric acid as a result of the adsorbent extraction, is used for preliminary cleaning of the outgoing air, containing ethylene oxide.

6. Process according to claim 5, characterised in that pure water, which reacts in the adsorbent bed with ethylene oxide to form glycol, is obtained by evaporating the water from the glycol solution, which is obtained from the preliminary cleaning of the outgoing air.

7. Process according to claim 6, characterised in that the liquid, which has become more concentrated as a result of water evaporation, is neutralised with calcium-containing chemicals.

8. Process according to claim 7, characterised in that the hydrated calcium sulphate formed is separated off and the neutralised, glycol-containing liquid is subjected to biological degradation.

9. Process according to claim 8, characterised in that the ethylene-oxide free outgoing air is used to aerate the process of biological glycol degradation.

## Revendications

1. Procédé d'élimination d'oxyde d'éthylène de courants d'air d'évacuation ou de gaz d'échappement, en formant du glycol à partir d'oxyde d'éthylène et d'eau en présence d'un acide comme catalyseur, caractérisé en ce que.
a) le courant de gaz à épurer humidifié à l'eau est acheminé à travers un lit d'un adsorbant, qui contient le catalyseur,
b) on utilise un liquide pour extraire le glycol de l'adsorbant, et
c) l'adsorbant extrait est séché et rechargé de catalyseur avant qu'il ne soit encore utilisé pour l'épuration des gaz d'échappement.

2. Procédé selon la revendication 1, caractérisé en ce qu'on utilise comme adsorbant du charbon actif granulaire.

3. Procédé selon la revendication 1, caractérisé en ce qu'on utilise comme catalyseur de l'acide sulfurique.

4. Procédé selon la revendication 1, caractérisé en ce qu'on utilise de l'eau comme agent d'extraction.

5. Procédé selon la revendication 4, caractérisé en ce qu'on utilise l'eau, chargée en glycol et en acide sulfurique par l'extraction de l'adsorbant, pour pré-épurer l'air d'évacuation contenant de l'oxyde d'éthylène.

6. Procédé selon la revendication 5, caractérisé en ce qu'on obtient de l'eau pure, qui réagit avec l'oxyde d'éthylène dans le lit de l'adsorbant pour former du glycol, par évaporation de l'eau de la solution de glycol, qui est produite lors de la pré-épuration de l'air d'évacuation.

7. Procédé selon la revendication 6, caractérisé en ce qu'on neutralise le liquide concentré par évaporation de l'eau par utilisation de produits chimiques contenant du calcium.

8. Procédé selon la revendication 7, caractérisé en ce qu'on élimine le gypse formé et on soumet le liquide neutralisé contenant du glycol à une décomposition biologique.

9. Procédé selon la revendication 8, caractérisé en ce que l'air d'évacuation débarrassé de l'oxyde d'éthylène est utilisé pour alimenter en air la décomposition biologique du glycol.
